# EUROPÄISCHE PATENTSCHRIFT

(11) **EP 2 663 257 B1**
(45) Veröffentlichungstag und Bekanntmachung des Hinweises auf die Patenterteilung: **26.10.2016**
(21) Anmeldenummer: 11700726.0
(22) Anmeldetag: 11.01.2011
(51) Int. Cl.: A61F 2/24

(54) **KLAPPENPROTHESE ZUM ERSATZ EINER ATRIOVENTRICULARKLAPPE DES HERZENS**
VALVE PROSTHESIS FOR REPLACING AN ATRIOVENTRICULAR VALVE OF THE HEART
PROTHÈSE VALVULAIRE DESTINÉE À REMPLACER UNE VALVULE AURICULO-VENTRICULAIRE DU COEUR

(43) Veröffentlichungstag der Anmeldung: 20.11.2013
(73) Patentinhaber: Figulla, Hans Reiner, 07749 Jena (DE); Lauten, Alexander, 14532 Kleinmachnow (DE)
(72) Erfinder: Figulla, Hans Reiner, 07749 Jena (DE); Lauten, Alexander, 14532 Kleinmachnow (DE)
(74) Vertreter: Trinks, Ole
(86) Internationale Anmeldenummer: PCT/EP2011/000082
(87) Internationale Veröffentlichungsnummer: WO 2012/095116

(56) Entgegenhaltungen:
- WO-A1-2010/057262
- WO-A2-03/037227
- US-A1- 2007 050 020

## Beschreibung

Die Erfindung betrifft eine Klappenprothese zum Ersatz einer Atrioventricularklappe des Herzens, also der Mitralklappe oder der Trikuspidalklappe.

Bei Patienten mit einer Funktionsbeeinträchtigung einer Herzklappe ist ein offener chirurgischer Eingriff am Herzen zum Einsatz einer Klappenprothese (Ersatzklappe) aufgrund des Allgemeinzustandes des Patienten häufig mit erhöhten Risiken verbunden, sodass Herzklappenprothesen zunehmend minimal invasiv über einen Katheter implantiert werden.

Der Stand der Technik kennt hierfür den Einsatz von Stents, in denen Ersatz-Herzklappensegel befestigt sind und die zur Einführung über einen Katheter stark komprimierbar sind und so durch den Katheter an den Ort der zu ersetzenden Herzklappe vorschiebbar und dort freisetzbar sind. Der zum Beispiel ballonexpandierbare oder selbstexpandierbare Stent entwickelt im freigesetzten Zustand eine radiale Spreizkraft, die eine Verankerung der Ersatz-Klappenprothese bewirkt oder zumindest fördert. Für eine solche Verankerung der Klappenprothese ist eine Ersatz-Aortenklappe besonders geeignet, die mit radialer Spreizkraft am Ort der dysfunktionalen Aortenklappe verankert werden kann. Zum Stand der Technik hierzu vgl. z.B. EP 1 994 913 A2; EP 1 469 797 B1; EP 1 259 195 B1; WO 2007/051620 A1; WO 2007/048529 A1; EP 1 980 220 A1; WO 01/64137 A1; EP 1 255 510 B3; und US 5,411,552.

Herzklappen mit Verankerungsteilen sind aus der WO2010/057262A1 und US2007/0050020 A1 bekannt.

Die Mitralklappe des Herzens, also die Klappe zwischen dem linken Vorhof (Atrium) und der linken Kammer (Ventrikel) ist allerdings wenig geeignet für eine Ersatzprothese, die wesentlich durch radiale Spreizkraft eines Stents kraftschlüssig (durch Reibung) vor Ort verankert ist, da eine Aufweitung des Annulus zu vermeiden ist.

Der Erfindung liegt die Aufgabe zu Grunde, eine Klappenprothese zum Ersatz einer Atrioventricularklappe bereitzustellen, die mittels eines Katheters implantierbar ist und eine stabile sowie orthotrope Positionierung und Verankerung ermöglicht.

Die Herzklappenprothese der Erfindung ist in den Ansprüchen definiert.

Erfindungsgemäß erfolgt die Verankerung der Klappenprothese mittels hakenartiger Verankerungsteile, die fest mit einem Ringkörper verbunden sind, an dem oder in dem die Herzklappensegel (Ventilblättchen) befestigt sind. Die Verankerungsteile erstrecken sich vom Ringkörper parallel zur Achse desselben in den Ventrikel hinein und sind so bemessen, dass sie mit einem am ventrikelseitigen Ende des Verankerungsteils vorgesehenen Haken im dort gegebenen Gewebe, wie insbesondere Sehnenfäden, Papillarmuskeln oder Klappensegeln verhakt werden können. Es sind also die beiden Funktionen der Klappenprothese, nämlich einerseits die Ersatz-Herzklappensegel abzustützen und zu halten, und andererseits die Verankerung der Klappenprothese, räumlich voneinander getrennt: Der Ringkörper wird im Annulus positioniert und hält dort die Ersatz-Herzklappensegel, während die Verankerungsteile in der vorstehend beschriebenen Weise unter Abstand vom Ringkörper die Verankerung der Klappenprothese bewirken.

Der Ringkörper ist bevorzugt etwa diaboloförmig ausgestaltet, sodass der Annulus in einen mittigen Abschnitt des Ringkörpers, der einen im Vergleich zu seinen Enden reduzierten Durchmesser aufweist, eingreifen kann. Dabei ist der Ringkörper hinsichtlich seiner Abmessungen und seiner elastischen Eigenschaften so gestaltet, dass bei Einsatz im Klappenannulus des Herzens keine den Klappenannulus wesentlich aufweitenden radialen Spreizkräfte wirken.

In der Systole ist der Ventrikel gegen das Atrium abzudichten. Diese Abdichtung leistet die Ersatz-Klappenprothese. Die dabei auf die Herzklappensegel der Prothese wirkende Kraft entspricht dem vollen Blutdruck. Gegen diese Kraft muss die Klappenprothese stabil und dauerhaft orthotrop verankert werden. Die Erfindung nutzt die Erkenntnis, dass diese Kraft im Wesentlichen in Richtung der zentralen Achse des Ringkörpers (entsprechend in Richtung der zentralen Achse des Annulus) wirkt, sodass die Verankerung entsprechende Zugkräfte in dieser Richtung auffangen muss. Deshalb kann die erfindungsgemäße Klappenprothese darauf verzichten, dass wesentliche Verankerungskräfte durch Reibschluss zwischen der Klappenprothese und umliegendem Gewebe erzeugt werden. Vielmehr wird die Klappenprothese während der Systole, in der sie den Schließzustand einnimmt, über in axialer Richtung wirkende Zugkräfte gehalten, die über die Verankerungsteile in das umliegende Gewebe, also insbesondere die Sehnenfäden eingeleitet und dort aufgefangen werden.

Die Verankerungsteile sind hierzu mit hakenartigen Verankerungsteilkomponenten versehen, zum Beispiel derart, dass das Verankerungsteil insgesamt etwa die Gestalt eines Winkelstückes hat mit einer sich im Wesentlichen axial erstreckenden armartigen Komponente, die an ihrem atriumseitigen Ende mit dem Ringkörper fest und insbesondere drehfest verbunden ist, und mit einer am ventrikelseitigen Ende des Armes angeordneten Komponente, die um die Achse des Ringkörpers herum gekrümmt ist und deshalb bei Drehung der Verankerungsteile mit Gewebe verhakt werden kann, um die Klappenprothese insgesamt stabil zu verankern, insbesondere gegenüber ventrikelseitigem Druck.

Für eine Mitralersatzklappe empfiehlt die Erfindung zwei diametral einander gegenüberliegende Verankerungsteile, während für eine Trikuspidalklappe drei Verankerungsteile vorgeschlagen werden, die den Vollkreis in Abschnitte von 120° aufteilen.

Der Ringkörper ist bevorzugt aus einem Drahtgeflecht geformt, insbesondere aus einem Material mit Formgedächtnis.

Die Herzklappensegel als solche können gemäß dem Stand der Technik am oder im Ringkörper befestigt werden, z. B. gemäß US 5 411 552 oder EP 1 255 510 B3. Zum Beispiel kann eine von einem Schwein gewonnene Mitralklappe oder Trikuspidalklappe in den Ringkörper eingenäht werden.

Die Erfindung stellt eine Herzklappenprothese bereit, deren ventrikelseitige Verankerung eine stabile und orthotrope Positionierung ermöglicht. Die Verankerung verhindert eine Verschiebung der Klappenprothese in axialer Richtung. Wenn hier von axialer oder radialer Richtung sowie Umfangsrichtung gesprochen wird, bezieht sich dies auf den genannten Ringkörper der Klappenprothese beziehungsweise, im implantierten Zustand, auf den Annulus der ersetzten Mitral- bzw. Trikuspidalklappe.

Die erfindungsgemäße Atrioventricularersatzklappe ist ballonexpandierbar oder selbstexpandierbar.

Implantierbar ist die Ersatzklappe mittels eines Katheters transepital, transapikal über die Herzspitze, oder retrograd durch die Aorta.

Die Positionierung der Klappenprothese mittels eines Katheters erfolgt bevorzugt derart, dass die beschriebenen Verankerungsteile als erstes am Katheterende freigesetzt werden und sodann mit ihren Haken im Gewebe verankert werden, insbesondere durch Drehung der gesamten Klappenprothese mitsamt der Verankerungsteile um die Achse. Sodann wird die Klappenprothese vollständig auch mit dem Ringkörper freigesetzt, derart, dass der Ringkörper mit seiner Mitte etwa auf dem Annulus der ersetzten Klappe zu liegen kommt.

In Abwandlung des vorstehend beschriebenen Ausführungsbeispieles und dabei nicht Teil der Erfindung, können die Verankerungsteile auch so gestaltet werden, dass ihre hakenförmigen Verankerungsteilkomponenten nicht in Umfangsrichtung verlaufen, sondern radial nach Außen weisende Haken aufweisen, die beim Freisetzen der Klappenprothese aus dem Katheter radial nach Außen aufgespreizt werden, sodass eine Verankerung in den Sehnenfäden oder anderem Gewebe, wie Segeln (Ventilblättchen) der nativen Atrioventricularklappen ermöglicht ist.

Die efindungsgemäßen Verankerungsteile sind so gestaltet, dass zwischen ihnen im Ventrikel der Zugang zur Aorta und der Aortenklappe für eine ungehinderte Blutströmung frei bleibt.

Eine weitere bevorzugte Ausgestaltung der Erfindung sieht vor, dass die axiale Länge der Verankerungsteile einstellbar ist, z. B. über eine arretierbare Teleskopanordnung, eine Schraubverbindung oder dergleichen, sodass der Chirurg vor Ort eine Anpassung an die anatomischen Gegebenheiten vornehmen kann.

Wenn hier von Sehnenfäden die Rede ist, dann umfasst dies auch die flächigen Verbindungsstücke derselben nahe den Segeln der Klappe.

Nachfolgend werden Ausführungsbeispiele der Erfindung anhand der Zeichnung näher erläutert. Es zeigt:
- Figur 1: schematisch eine Klappenprothese zum Ersatz einer Atrioventricularprothese des Herzens;
- Figur 2: eine Klappenprothese gemäß Figur 1 in implantiertem Zustand im Herzen; und
- Figur 3: eine Klappenprothese in vergrößertem Maßstab.

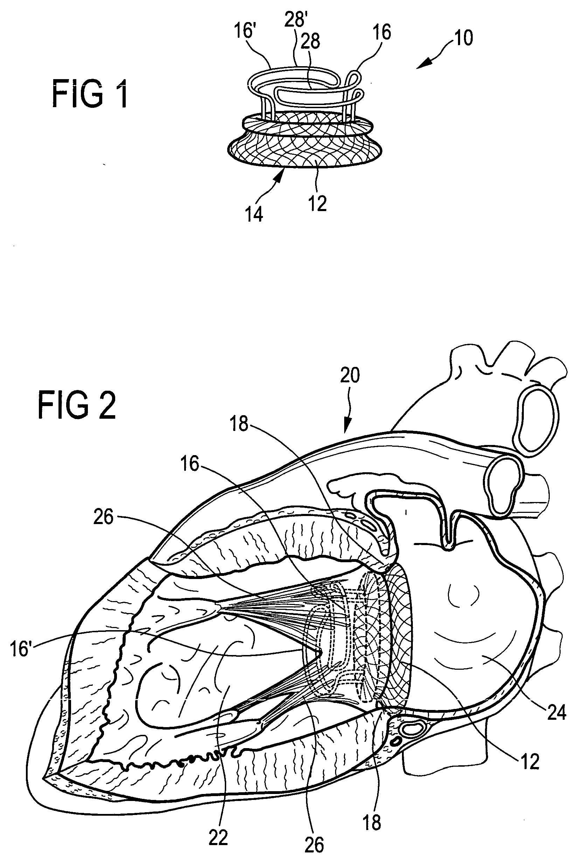

Die in Figur 1 gezeigte Klappenprothese 10 zum Ersatz einer Atrioventricularklappe des Herzens weist einen Ringkörper 12 auf, in dem nicht näher gezeigte Herzklappensegel befestigt sind. Für die Herzklappensegel 14 können als solches bekannte Systeme des Standes der Technik eingesetzt werden, z. B. Segel aus Pericardium, oder z. B. vom Herz eines Schweins gewonnene Herzklappen, die in dem Ringkörper 12 vernäht werden. Der Ringkörper 12 ist aus einem Drahtgeflecht gebildet und die elastischen Eigenschaften des Drahtes und die Abmessungen des Ringkörpers 12 sind so gewählt, dass der Ringkörper im Einsatz der Klappenprothese als Mitralklappe oder Trikuspidalklappe - anders als üblicherweise ein Stent - keine den natürlichen Klappenannulus wesentlich aufweitende radiale Spreizkraft erzeugt.

Für das Drahtgeflecht des Ringkörpers 12 kann ein Metall mit Formgedächtnis eingesetzt werden, z. B. Nitinol.

Wie die Figuren 1, 2 und 3 zeigen, ist der Ringkörper rotationssymmetrisch um seine Achse A. Der atriumseitige Durchmesser D1 des Ringkörpers 12 ist größer als der ventrikelseitige Durchmesser D2 und dieser ist wiederum größer als ein mittiger Durchmesser D3 des Ringkörpers 12.

Wie die Figuren zeigen, stehen vom Ringkörper 12 ventrikelseitig Verankerungsteile 16, 16' in axialer Richtung vor. Wie Figur 2 zeigt, ragen diese Verankerungsteile 16, 16' in den Ventrikel 22. Der Ringkörper 12 wird mit seinem mittleren Abschnitt, also dem Abschnitt mit reduziertem Durchmesser D3, im Annulus 18 der Mitralklappe positioniert, Figur 2 zeigt also den Einsatz der Klappenprothese zwischen linkem Atrium und linkem Ventrikel. Der Bereich des Ringkörpers 12 mit vergrößertem Durchmesser D1 liegt satt am atriumseitigen Abschnitt des Annulus 18 an und fixiert die Klappenprothese in ihrem Offenzustand bei Blutströmung aus dem Atrium 24 in das Ventrikel 22.

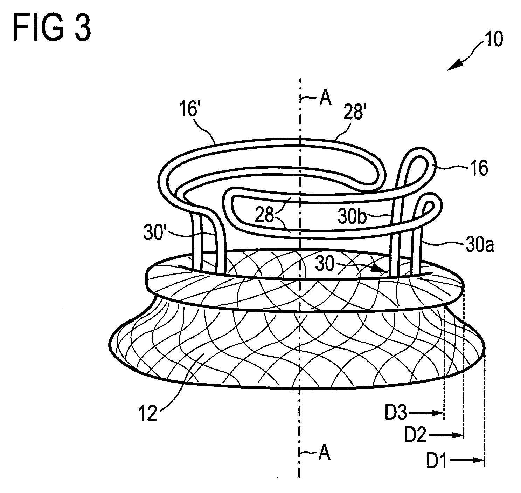

Die Verankerungsteile 16, 16' weisen beim Ausführungsbeispiel jeweils zwei Komponenten auf, nämlich zum einen Arme 30a, 30b, die sich im Wesentlichen parallel zur Achse A des Ringkörpers 12 erstrecken, und Komponenten 28, 28', die sich hakenartig um die Achse A des Ringkörpers 12 gekrümmt in Umfangsrichtung des Ringkörpers 12 erstrecken, wie Figur 3 im Einzelnen zeigt.

Beim dargestellten Ausführungsbeispiel hat ein Arm 30 zur drehfesten Verbindung von Verankerungsteil 16 mit Ringkörper 12 zwei Armkomponenten 30a, 30b, die sich jeweils im Wesentlichen parallel zur Achse A des Ringkörpers erstrecken und an ihren ventrikelseitigen Enden über schlaufenartige Biegungen in die sich in Umfangsrichtung erstreckenden Komponenten 28, 28' der Verankerungsteile übergehen. Die Verankerungsteile 16, 16' sind aus einem geeignetem Metalldraht geformt.

Die Verankerungsteile 16 sind insbesondere, je nach der gegebenen anatomischen Situation, so bemessen, dass der Abstand vom Annulus (18) zum ventrikelseitigen Ende des Verankerungsteils im Bereich von 5 bis 40 mm liegt, bevorzugt im Bereich von 5 bis 20 mm oder im Bereich von 10 bis 25 mm.

Ähnlich wie eine Stent-Klappenprothese ist die beschriebene Klappenprothese stark komprimierbar und über einen Katheter minimal-invasiv im Herzen positionierbar, nämlich insbesondere transepital, transapikal über die Herzspitze, oder auch retrograd über die Aorta. Hierfür können die als solche dem Fachmann bekannten Katheter-Techniken eingesetzt werden.

Das Verfahren zum Einsetzen der beschriebenen Klappenprothese sieht vor, dass die Verankerungsteile 16, 16' als erstes am Einsatzort aus dem Katheter freigesetzt werden. Beim in den Figuren 1 bis 3 dargestellten Ausführungsbeispiel erfolgt dann die Verankerung der Verankerungsteile 16' durch Drehung des Ringkörpers 12 mit den daran drehfest befestigten Verankerungsteilen 16, 16', welche sich dann mit ihren Komponenten 28, 28' z. B. in den Sehnenfäden oder auch in der nativen Seminularklappe verhaken. Die Drehung kann entweder durch Drehung des Katheters selbst erfolgen oder auch durch einen Pusher im Katheter, der einen Dreheingriff mit dem Ringkörper 12 erlaubt. Nach dieser Verhakung und Verankerung der Verankerungsteile im Gewebe, z. B. der Sehnenfäden oder der nativen Seminularklappe, erfolgt dann die vollständige Freisetzung der Klappenprothese aus dem Katheter, wobei der diaboloförmige Ringkörper mit seinem mittleren Bereich reduzierten Durchmessers gemäß Figur 2 im Annulus 18 fixiert wird.

In Abwandlung des vorstehend beschriebenen Ausführungsbeispieles und dabei nicht Teil der Erfindung können die Verankerungskomponenten 28, 28' dahingehend abgewandelt werden, dass sie sich nicht in Umfangsrichtung erstrecken, sondern radial nach Außen, sodass sie durch Aufspreizung nach dem Freisetzen in Verankerungseingriff mit den Sehnenfäden bringbar sind oder auch in Verankerungseingriff mit nativen Segeln, je nach den anatomischen Gegebenheiten.

## Patentansprüche

1. Klappenprothese zum Ersatz einer Atrioventricularklappe des Herzens mit
- einem Ringkörper (12), an dem Herzklappensegel (14) befestigt sind und der adaptiert ist, um in einen Klappenannulus (18) des Herzens (20) eingesetzt zu werden,
und mit
- zumindest einem Verankerungsteil (16, 16') hakenförmiger Gestalt, das vom Ringkörper (12) in Richtung des Ventrikels vorsteht und adaptiert ist, in Sehnenfäden (26), Papillarmuskeln oder Klappensegeln der Atrioventricularklappen verankert zu werden,
**dadurch gekennzeichnet, dass**
das zumindest eine Verankerungsteil einen Arm (30, 30a, 30b; 30') aufweist, der sich im Wesentlichen parallel zur Achse (A) des Ringkörpers (12) erstreckt, und eine Verankerungsteilkomponente (28, 28'), die sich frei in Umfangsrichtung erstreckt derart, dass sie bei Drehung des Ringkörpers (12) um dessen Achse (A) in verankernden Eingriff mit Gewebe, insbesondere Sehnenfäden bringbar ist.

2. Klappenprothese gemäß Anspruch 1 mit ein, zwei oder drei Verankerungsteilen (16, 16').

3. Klappenprothese gemäß einem der Ansprüche 1 oder 2, **dadurch gekennzeichnet, dass** der Ringkörper (12) atrialseitig einen im Vergleich zu einem mittigen Ringdurchmesser vergrößerten Durchmesser hat.

4. Klappenprothese nach einem der vorhergehenden Ansprüche, **dadurch gekennzeichnet, dass** der Ringkörper (12) ventrikelseitig einen im Vergleich zu einem mittigen Ringdurchmesser vergrößerten Durchmesser hat.

5. Klappenprothese nach den Ansprüchen 3 und 4, **dadurch gekennzeichnet, dass** der atrialseitige Durchmesser größer ist als der ventrikelseitige Durchmesser.

6. Klappenprothese nach einen der vorhergehenden Ansprüche, **dadurch gekennzeichnet, dass** das Verankerungsteil (16) adaptiert ist, im Schließzustand der Klappenprothese im Wesentlichen axiale Kräfte in Sehnenfäden (26) abzuleiten.

7. Klappenprothese nach einem der vorhergehenden Ansprüche, **dadurch gekennzeichnet, dass** der Ringkörper (12) adaptiert ist, bei Einsatz im Klappenannulus (18) des Herzens (20) keine den Klappenannulus wesentlich aufweitende radiale Spreizkraft zu erzeugen.

8. Klappenprothese nach einem der vorhergehenden Ansprüche, **dadurch gekennzeichnet, dass** für eine Mitralersatzklappe zwei Verankerungsteile vorgesehen sind.

9. Klappenprothese nach einem der vorhergehenden Ansprüche, **dadurch gekennzeichnet, dass** für eine Trikuspidalklappe drei Verankerungsteile vorgesehen sind.

10. Klappenprothese nach einem der vorhergehenden Ansprüche, **dadurch gekennzeichnet, dass** der Ringkörper (12) aus einem Geflecht geformt ist, insbesondere aus einem Material mit Formgedächtnis.

11. Klappenprothese nach einem der vorhergehenden Ansprüche, **dadurch gekennzeichnet, dass** der Ringkörper (12) ballonexpandierbar ist.

12. Klappenprothese nach einem der Ansprüche 1 bis 10, **dadurch gekennzeichnet, dass** der Ringkörper (12) selbstexpandierbar ist.

13. Klappenprothese nach einem der vorhergehenden Ansprüche, **dadurch gekennzeichnet, dass** die Länge eines Verankerungsteils (16) in axialer Richtung einstellbar ist.

## Claims

1. A prosthetic valve for replacing an atrioventricular heart valve comprising
- an annular body (12) to which valvular leaflets (14) are attached and which is adapted to be implanted in a valve annulus (18) of the heart (20),
and comprising
- at least one hook-shaped anchoring element (16, 16') protruding from the annular body (12) toward the ventricle and adapted to be anchored in the chordae tendineae (26), papillary muscles or leaflets of the atrioventricular valves,
**characterized in that**
the at least one anchoring element comprises an arm (30, 30a, 30b; 30') extending substantially parallel to the axis (A) of the annular body (12) and an anchoring element component (28, 28') freely extending in the circumferential direction such that it can be brought into anchoring engagement with tissue, particularly the chordae tendineae, upon rotation of the annular body (12) about its axis (A).

2. The prosthetic valve according to claim 1, having one, two or three anchoring elements (16, 16').

3. The prosthetic valve according to claim 1 or 2, **characterized in that** the atrial side of the annular body (12) is of larger diameter than its central diameter.

4. The prosthetic valve according to any one of the preceding claims, **characterized in that** the ventricular side of the annular body (12) is of larger diameter than its central diameter.

5. The prosthetic valve according to claim 3 and 4, **characterized in that** the atrial side is of larger diameter than the ventricular side.

6. The prosthetic valve according to any one of the preceding claims, **characterized in that** the anchoring element (16) is adapted to transfer substantially axial forces to the chordae tendineae (26) in the closed state of the prosthetic valve.

7. The prosthetic valve according to any one of the preceding claims, **characterized in that** the annular body (12) is adapted so as not to produce any substantially radial expanding force on the valve annulus when implanted in the valve annulus (18) of the heart (20).

8. The prosthetic valve according to any one of the preceding claims, **characterized in that** two anchoring elements are provided for a replacement mitral valve.

9. The prosthetic valve according to any one of the preceding claims, **characterized in that** three anchoring elements are provided for a replacement tricuspid valve.

10. The prosthetic valve according to any one of the preceding claims, **characterized in that** the annular body (12) is formed from a meshwork, particularly from a material having shape memory.

11. The prosthetic valve according to any one of the preceding claims, **characterized in that** the annular body (12) is balloon-expandable.

12. The prosthetic valve according to any one of claims 1 to 10, **characterized in that** the annular body (12) is self-expandable.

13. The prosthetic valve according to any one of the preceding claims, **characterized in that** the length of an anchoring element (16) is adjustable in the axial direction.

## Revendications

1. Prothèse valvulaire destinée à remplacer une valvule auriculoventriculaire du coeur, comportant
- un corps annulaire (12) sur lequel sont fixés des feuillets valvulaires (14) et qui est adapté à être mis en place dans un annulus valvulaire (18) du coeur (20),
et comportant
- au moins une pièce d'ancrage (16, 16') en forme de crochet, qui fait saillie du corps annulaire (12) en direction du ventricule et qui est adaptée à être ancrée dans des fibres ligamenteuses (26), dans des muscles papillaires ou dans des feuillets des valvules auriculoventriculaires,
**caractérisée en ce que**
ladite au moins une pièce d'ancrage comprend un bras (30, 30a, 30b ; 30') qui s'étend sensiblement parallèlement à l'axe (A) du corps annulaire (12), et un composant partiel d'ancrage (28, 28') qui s'étend librement en direction périphérique de telle sorte que lors d'une rotation du corps annulaire (12) autour de son axe (A), il est susceptible d'être amené en engagement d'ancrage avec du tissu, en particulier avec les fibres ligamenteuses.

2. Prothèse valvulaire selon la revendication 1, comportant une, deux ou trois pièces d'ancrage (16, 16').

3. Prothèse valvulaire selon l'une des revendications 1 ou 2, **caractérisée en ce que** le corps annulaire (12) présente du côté atrial un diamètre agrandi par comparaison à un diamètre central.

4. Prothèse valvulaire selon l'une des revendications précédentes, **caractérisée en ce que** le corps annulaire (12) présente du côté ventricule un diamètre agrandi par comparaison à un diamètre central.

5. Prothèse valvulaire selon l'une des revendications 3 et 4, **caractérisée en ce que** le diamètre côté atrial est supérieur du diamètre côté ventricule.

6. Prothèse valvulaire selon l'une des revendications précédentes, **caractérisée en ce que** la pièce d'ancrage (16) est adaptée pour dissiper, dans l'état de fermeture de la prothèse valvulaire, des forces sensiblement axiales vers les fibres ligamenteuses (26).

7. Prothèse valvulaire selon l'une des revendications précédentes, **caractérisée en ce que** le corps annulaire (12) est adapté pour ne pas produire, lors de la mise en place dans l'annulus valvulaire (18) du coeur (20), de force d'écartement radiale qui ferait sensiblement élargir l'annulus valvulaire.

8. Prothèse valvulaire selon l'une des revendications précédentes, **caractérisée en ce qu'**il est prévu deux pièces d'ancrage pour une valve mitrale de remplacement.

9. Prothèse valvulaire selon l'une des revendications précédentes, **caractérisée en ce qu'**il est prévu trois pièces d'ancrage pour une valve tricuspide.

10. Prothèse valvulaire selon l'une des revendications précédentes, **caractérisée en ce que** le corps annulaire (12) est formé d'une tresse, en particulier en un matériau à mémoire de forme.

11. Prothèse valvulaire selon l'une des revendications précédentes, **caractérisée en ce que** le corps annulaire (12) est expansible au moyen d'un ballon.

12. Prothèse valvulaire selon l'une des revendications 1 à 10, **caractérisée en ce que** le corps annulaire (12) est autoexpansible.

13. Prothèse valvulaire selon l'une des revendications précédentes, **caractérisée en ce que** la longueur d'une pièce d'ancrage (16) est réglable en direction axiale.
